# EUROPEAN PATENT SPECIFICATION

(11) **EP 3 463 552 B1**
(45) Date of publication and mention of the grant of the patent: **27.05.2020**
(21) Application number: 17728178.9
(22) Date of filing: 02.06.2017
(51) Int. Cl.: A61N 1/04, A61N 1/32

(54) **ELECTRICAL DEVICE WITH DEFORMABLE RESERVOIR AND CONDUCTOR**
ELEKTRISCHE VORRICHTUNG MIT VERFORMBAREM BEHÄLTER UND LEITER
DISPOSITIF ÉLECTRIQUE PRÉSENTANT UN RÉSERVOIR DÉFORMABLE ET UN CONDUCTEUR

(30) Priority: 02.06.2016 FR 1655031
(43) Date of publication of application: 10.04.2019
(73) Proprietor: L'Oréal, 75008 Paris (FR)
(72) Inventor: PLANARD-LUONG, Thi Hong Lien, 94152 Chevilly La rue (FR)
(74) Representative: L'Oreal
(86) International application number: PCT/EP2017/063522
(87) International publication number: WO 2017/207784

(56) References cited:
- FR-A1- 2 980 370
- FR-A1- 3 015 300

## Description

The present invention relates to devices for carrying out a cosmetic treatment of keratin materials.

More generally, a cosmetic product is a product as defined in Regulation (EC) No 1223/2009 of the European Parliament and Council of 30 November 2009 relating to cosmetic products.

The invention relates in particular to devices for treating keratin materials with the aid of an electric current.

The expression *"human keratin materials"* is understood as meaning mainly the skin, notably of the body or of the face, or the scalp, the nails or the hair.

### Technological background

It is known that the application of an electric current to the skin can facilitate the penetration of an active agent. It is thus known to treat human keratin materials with the aid of iontophoretic devices. Iontophoresis allows the diffusion of active agents through the skin by virtue of electrical stimulation in a non-invasive manner. The current administered can be adjustable in terms of intensity and polarity (anodic or cathodic current). The transcutaneous diffusion of the molecules via iontophoresis is based on two principles, namely electrorepulsion and electroosmosis.

Electrorepulsion is the migration of an ionized molecule by repulsion of charges of the same sign. Thus, a positively charged substance will diffuse through the skin at the anode (+).

Electroosmosis is the migration of a molecule, even a non-ionized molecule, by entrainment associated with the flow of water from the anode to the cathode during iontophoresis. The migration is due in particular to the negative charge of the skin. Under the effect of a current, dissolved substances are entrained by the water or a solvent during migration.

The patent US 5 090 402 relates to an electric current applicator that also has a massaging function, comprising a plurality of balls, all the balls being immersed in the composition reservoir which extends under a ball holder.

The patent application FR 2 980 370 discloses an electrostimulation device to be applied on an area of the skin, wherein an electric current is supplied by electrodes. The device comprises a reservoir containing the cosmetic product to be dispensed and a distribution channel. A dispensing electrode supplies the cosmetic product through an outlet orifice to be applied on the skin.

The patent application WO2005115281 discloses an ocular iontophoretic system comprising an active electrode, a reservoir and active agents stored in the reservoir, a passive electrode for looping the electrical circuit, and a power supply that supplies a current to the electrodes. The active electrode can be disposed in the reservoir by fitting it therein or by forming it directly therein (for example by electrodeposition). The active electrode can be produced by coating a conductive wire with a conductive layer, for example in the form of a grid or network. The active electrode is optionally flexible enough to be able to deform under the action of mechanical forces such as those which are exerted during the application of the device to the eye. The reservoir may comprise flexible side walls that are formed for example from silicone, this material being very suitable for ocular contact. The reservoir may comprise feed tubes for the active agent and possibly outlet tubes for active agent.

According to said document WO2005115281, the cosmetic product is in a compact and solid form. It comes directly into static contact with the eye or with the eyelid, protruding from the reservoir between two lips. However, it is not very suitable for dynamic treatment with a liquid composition because there is a risk of the composition leaking.

There is a need to further improve devices for cosmetic treatment with the aid of an electric current which facilitate the penetration of active agents so as to increase the efficacy of iontophoresis.

There is a need to benefit from a device for treating keratin materials with the aid of an electric current, which exhibits good efficacy and can be used with comfort and in complete safety, including for dynamic treatment.

There is also a need to benefit from such a device that comprises a reservoir and provides regular distribution of the product over the surface to be treated, so as to bring about uniform care over this surface without favouring particular areas.

### Definition of the invention

Therefore, the subject of the invention, according to one of its aspects, is a device for cosmetically treating keratin materials with the aid of an electric current, comprising at least:
- a power supply system,
- an electrode and a counter electrode,
- a removable reservoir containing a cosmetic composition, the reservoir being provided with at least one outlet orifice for the composition,
- an application end piece that is fed with composition from the reservoir and defines an application surface intended to come into contact with the keratin materials,
the reservoir is at least partially defined by a first, electrically conductive zone that is connected to the power supply system, and by a second, elastically deformable zone, the second, elastically deformable zone exerting a pressure on the composition that is able to bring about a decrease in volume of the reservoir and a flow of the composition through the outlet orifice in the opened-up position of the outlet orifice, and the composition being kept inside the reservoir in the closed-off position of the outlet orifice.

According to the invention, the composition is released progressively from the reservoir as soon as the outlet orifice is opened up. Thus, the outlet flow of the composition is regulated in a simple manner, solely by the relaxation of the second, elastically deformable zone, which tends to return to its equilibrium state.

The nature of the elastically deformable wall can be chosen so as to cause a more or less rapid outlet flow of the composition out of the reservoir, depending on the intended treatment and the rheology of the composition. The flow rate can be easily controlled by this choice.

In addition, the composition can be distributed more homogeneously over the skin, as long as the user moves the device continuously. The efficacy of the cosmetic treatment is improved over the entire area treated.

The number of parts of the device can be reduced: there is no need for a motor or piston for evacuating the composition from the reservoir. All that is necessary to start it is to open up the outlet orifice.

The elastically deformable wall is able to move between a first position defining the maximum volume of the reservoir and a second position defining the minimum volume of the reservoir. The maximum volume of the reservoir is the volume of the reservoir in the closed position of the outlet orifice.

The presence of the elastically deformable wall has the effect of smoothing the distribution of the composition over the keratin materials.

According to a first embodiment of the invention, the first, electrically conductive zone is separate from the second, elastically deformable zone.

According to a second embodiment, the first, electrically conductive zone coincides with the second, elastically deformable zone, with an elastic and conductive material then being used.

### Reservoir

Advantageously, the reservoir is defined by a maximum volume of between 50 microlitres (µl) and 10 ml.

Depending on the volume chosen, the reservoir may be a single-dose reservoir or a multiple-use reservoir.

The device advantageously comprises a system for controlling the flow rate of the composition emerging from the reservoir to between 1 µl/cm² and 50 µl/cm².

The composition is dispensed at the outlet of the device continuously and without jolts. The regularity of the dispensing flow rate ensures the efficacy and the stability of the microcurrent applied and thus the comfort of the user during the treatment. More preferably, each outlet orifice defines an outlet cross-sectional area for the composition of less than 10 mm², preferably between 0.03 mm² and 10 mm².

The outlet orifices can be distributed regularly over the walls of the reservoir. For example, the reservoir may comprise a porous zone that defines the outlet orifices, for example a woven or nonwoven fabric.

The reservoir may be disposed in a housing situated inside the device. In this case, it may be necessary to open the device in order to have access to the reservoir.

The reservoir may be manufactured by injection blow moulding or extrusion blow moulding or welding of a preformed film.

The reservoir in particular may be a single-use reservoir . It may or may not be a single-dose reservoir.

The reservoir may also form a cushion disposed directly in contact with the application end piece, optionally accessible to the user from outside the device. It may be covered with an end piece formed by a woven fabric or a porous material.

Advantageously, the reservoir comprises an external layer of conductive hydrogel that is able to stabilize the pH.

### First electrically conductive zone

An *"electrically conductive material"* is a material that is able to allow electrical charges to move, that is to say to allow the passage of the electric current. Advantageously, the electrically conductive material has a conductivity greater than 1 mS/cm and better still greater than 100 mS/cm or more.

Advantageously, the first, electrically conductive zone forms an electrode. In this way, the number of parts of the device is reduced.

More advantageously, the first, electrically conductive zone is remote from the application surface. With this arrangement, burning or heating of the body surface is avoided or reduced.

According to one embodiment, the first, electrically conductive zone comprises a conductive coating or a conductive material. The user is provided with a significant number of possibilities for choosing the nature of the first zone, depending on the desired effect and the treatment to be carried out.

The connection to the power supply system has the effect of ensuring that the electric current is transferred from the power supply system to the reservoir or the electrode. The first zone, the electrode and the composition may be the only elements in the device that are electrically conductive. In particular, it is possible for the cap, the cover and/or the application members not to be electrically conductive. The only element which is in contact with the skin and is electrically conductive may be the composition.

### Second, elastically deformable zone

According to the invention, an *"elastically deformable material"* is a material that advantageously has a level of elongation of at least 2%.

Advantageously, the second, elastically deformable zone comprises an elastomer, notably a silicone elastomer. The elasticity of an elastomer is particularly suitable for compression of the reservoir that is appropriate for the desired flow rate.

More advantageously, the second, elastically deformable zone comprises a material that has a level of elongation of at least 10%, and preferably at least 20%, even more preferably 30%. These values are appropriate for progressive squeezing of the reservoir.

### Opening and closing of the outlet orifice

Before use, the outlet orifice can be closed off by a removable closure. The latter can be removed in the position releasing the orifice.

Even more advantageously, in the closed position of the outlet orifice, the reservoir comprises a frangible zone that is able to tear under the effect of an action by the user for opening up the outlet orifice. The user can, for example, exert an action so as to set a member, such as a screw, a tip or a blade that is able to break the frangible zone of the reservoir, into motion.

### Application end piece

The application end piece may or may not be conductive.

It may comprise several application members, notably in the form of balls or spikes. The multiplicity of application members facilitates good distribution of the composition in a continuous manner, and improves the ionization of the composition during the treatment, if appropriate. The multiplicity of application members also makes it possible to improve the massaging effect during treatment.

Advantageously, the application end piece comprises at least one ball that is movable about a centre of rotation.

As a variant, the application members may have any profile, notably a cylindrical profile, for example in the form of rollers, or a non-cylindrical profile, for example an ovoid or discoid shape.

The application members may be made of plastic or metal.

The application end piece may be a pad.

The outer surface of the end piece may be totally inert from a chemical point of view with regard to the composition applied and the keratin materials. The outer surface may be covered with a varnish. The outer surface may be polished. The outer surface may comprise a biocidal material, if need be.

### Power supply system

The device may comprise a power supply system for exposing the keratin materials to an electric treatment current in an application zone for the composition.

A *"power supply system"* is understood to be an electrical assembly that is able to induce a potential difference between one or more electrodes and at least one counter electrode. If the application end piece is placed on the face and if the counter electrode is held in one hand, the potential difference is established between the face and the hand.

Advantageously, the power supply system delivers an alternating current with frequencies of, preferably, between 1 Hz and 5 MHz.

### Electrodes

The power supply system may comprise an electrode located at a distance from the keratin materials and in contact with the composition, and a counter electrode that preferably comes into contact with the keratin materials in a zone that is not exposed to the composition. It may comprise a multi-electrode system, such as interlocking electrodes or an array. If an alternating current is used, the polarities of the electrode and of the counter electrode alternate.

According to one embodiment of the invention, an *"electrode"* is understood to mean a positively charged electrode (anode) or a negatively charged electrode (cathode). This electrode may be disposed in the application end piece, so as to ensure the passage of the electric current into the composition. The electrode is then disposed inside the end piece. In this case, it does not come directly into contact with the keratin materials, but with the composition itself. The composition may be the only conductive substance in contact with the skin while the device is being used. The electrode may not be in contact with the keratin materials, notably the skin, the end piece not comprising any electrically conductive material in contact with the skin.

Throughout the text, the term *"electrode"* means a single isolated electrode. An electrode may be in the form of a ball, spike, stud, tongue, for example. The device may comprise a single or several electrodes.

A "counter electrode" is understood to be an electrode that is charged with an opposite sign to the electrode: negative, positive or alternating or connected to the earth of the generator. In general, said counter electrode is disposed on the body of the device or on a handpiece. The counter electrode is intended to come into contact with an area of the body of the person undergoing the care. In one embodiment, the counter electrode is disposed on the end piece. If this is the case, it is separated from the electrode by an insulating space.

The electrode may be housed inside the end piece, the electrode notably being spaced apart from the outer wall of the application end piece by a distance of between 0.2 mm and 5 mm. This distance is the shortest measurable distance between the electrode and the outer surface of the application end piece. It is measured between any points of the electrode and of the outer surface of the application end piece, as long as the distance measured is the shortest distance.

Advantageously, the power supply system comprises a current or voltage generator that is able to control the intensity of the treatment current flowing between the electrode and the skin, thereby allowing the voltage U between the electrode and the counter electrode to be controlled and possibly allowing the intensity of the current to be controlled. The voltage U generated depends on the impedance of the *"skin* + *composition"* system.

The electrode may be flat, for example in the form of a flat disc or of a polygon. The electrode may be hollow, being formed for example by stamping or bending an electrically conductive metal sheet. The electrode may be porous. The electrode may be formed by a surface treatment for making the first zone conductive.

### Electrical parameters

The electrical power source may include any cell or any accumulator. The voltage between the electrodes is for example between 1.2 V and 24 V, preferably between 1.2 and 3.3 V. If appropriate, the passage of the current can create spot heating.

At equivalent current density, the device can notably deliver a current density, at the skin, of preferably less than or equal to 0.500 mA/cm² r.m.s., for example between 0.01 mA/cm² r.m.s. and 0.500 mA/cm² r.m.s., for example between 0.1 mA/cm² r.m.s. and 0.3 mA/cm² r.m.s.

### Complementary cosmetic treatments

Advantageously, the device comprises a source of heat, light, for setting the application end piece in motion, notably a sonic, ultrasonic, radiofrequency or electroporation vibration.

### Composition

Preferably, the cosmetic composition has a viscosity, measured at 25°C and at a normal atmospheric pressure of 1.013 10⁵ Pa, of less than 0.5 Pa.s, more preferably less than 0.3 Pa.s, better still less than 0.2 Pa.s, notably in the range from 0.1 to 0.2 Pa.s, better still from 0.04 to 0.175 Pa.s.

The viscosity of the composition is measured at 25°C and at a normal atmospheric pressure of 1.013 10⁵ Pa using a Rheomat 180 (from the company Lamy), equipped with an MS-R1, MS-R2, MS-R3, MS-R4 or MS-R5 spindle chosen depending on the consistency of the composition, and rotating at a rotation speed of 200 rpm. The measurement is taken after 10 minutes of rotation. The viscosity measurements are taken at most 1 week after production.

In the context of the invention, the spindles RS-R3 or MS-R2 or, for the most fluid compositions, MS-R1 will be used.

It is possible to use at least one cosmetic or dermatological composition with the device.

The composition(s) used may be in any form, for example in the form of an aqueous solution, an oil, an emulsion, a powder or a gel. The composition(s) used may also be sprayed onto the skin.

When the composition(s) used is/are in the form of a powder or gel, the latter can take on the shape of the electrode to which it is applied, as mentioned above. One or more compositions can be applied. To this end, the reservoir may comprise several compartments.

The composition(s) may comprise an active agent.

Advantageously, the composition is chosen from:
- a face care or body care composition, comprising in particular an active agent chosen from humectant or moisturizing active agents, anti-ageing active agents, for example, depigmenting active agents, active agents that act on cutaneous microcirculation or seboregulating, anti-acne, filling, vitamin active agents;
- a temporary, semi-permanent or permanent composition for making up the face or body,
- a hair composition, in particular a composition for washing the hair, for hair care or conditioning, for temporary form retention or shaping of the hair, for the temporary, semi-permanent or permanent dyeing of the hair, or for relaxing or permanent-waving, in particular a composition for relaxing, dyeing or bleaching the roots and hair,
- a composition for the scalp, in particular an antidandruff composition, a composition for preventing hair loss or for promoting regrowth of the hair, an anti-seborrhoeic, anti-inflammatory, anti-irritation or soothing composition, a mark-preventing composition or a composition for stimulating or protecting the scalp.

The device may be used in various cosmetic or dermatological treatments, for example for combating wrinkles, herpes, acne or for redensifying the skin or the hair.

### Description of the figures

The invention will be better understood from reading the following detailed description of non-limiting illustrative embodiments of the invention and from examining the appended, schematic and partial drawing, in which:
- Figure 1 is a perspective view of a single-dose reservoir intended to equip a device according to the invention,
- Figure 2 is a perspective view of a device according to the invention equipped with the reservoir in Figure 1,
- Figure 3 is a longitudinal section through the device in Figure 1,
- Figure 4 is a cross-sectional view AA of the single-dose reservoir in Figure 1,
- Figure 5 shows the detail C from Figure 3.

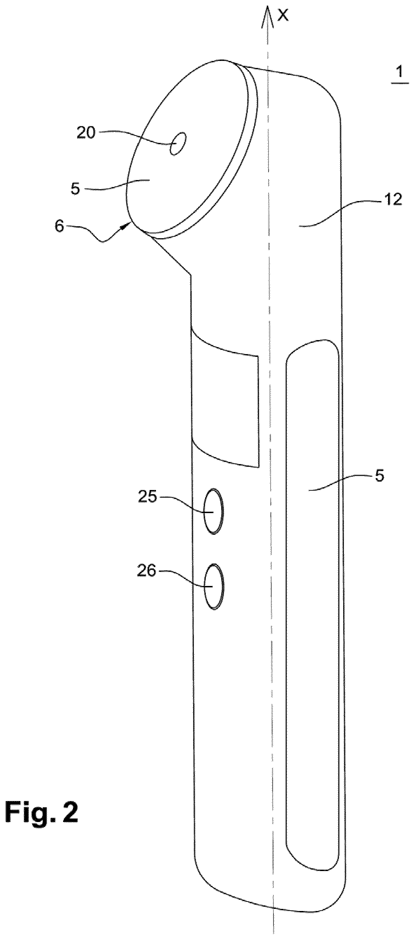

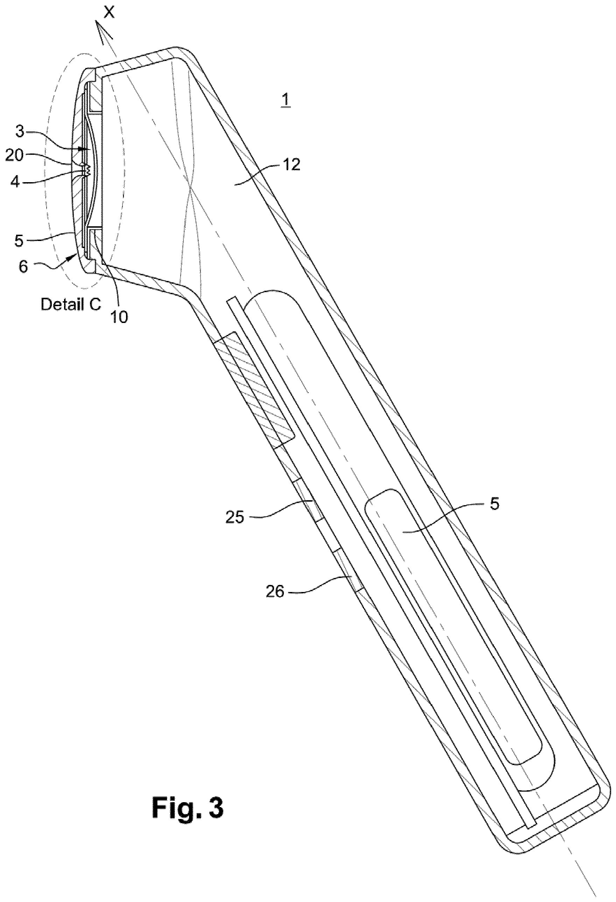

Figures 2 and 3 show a device 1, of axis X, in accordance with the invention. It comprises a body 12 on which an end piece 6 that defines a flat application surface 5 is mounted. A single-dose reservoir 3 of composition is housed inside the body 12 in contact with the application end piece 6.

In the example in question, the body 12 can be made of thermoplastic material. As a variant, it may be made of any other material. The application end piece 6 could have any other shape, for example that of a roller. It may be force-fitted, snap-fastened or screwed onto the body 12.

The device 1 comprises an electrode 10 housed between the body 12 and the end piece 6, and also a counter electrode 5 disposed on the body 12. The counter electrode 15 is in contact with the user's hand when the latter holds the device 1 in their hand, such that the electrical circuit between the electrode 10 and the counter electrode 15 is closed by passing through the user's body at the moment of treatment. The polarization of the electrode 10 and that of the counter electrode 15 may be reversible (+ or -) depending on the nature of the composition used.

The electrode 10 is connected to a pole of a power supply system housed in the body 12, and communicates with the reservoir 3 containing the composition. The composition then passes through the outlet orifice 4 of the reservoir 3. It then passes through a dispensing duct 20 in the end piece 6 in order to pass onto the application surface 5 and then onto the skin to be treated.

The counter electrode 15 is connected to the other pole of the power supply system, which is powered by a battery (cells or rechargeable battery), for example.

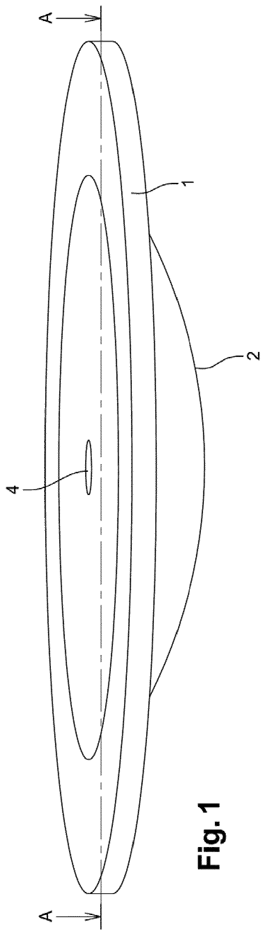

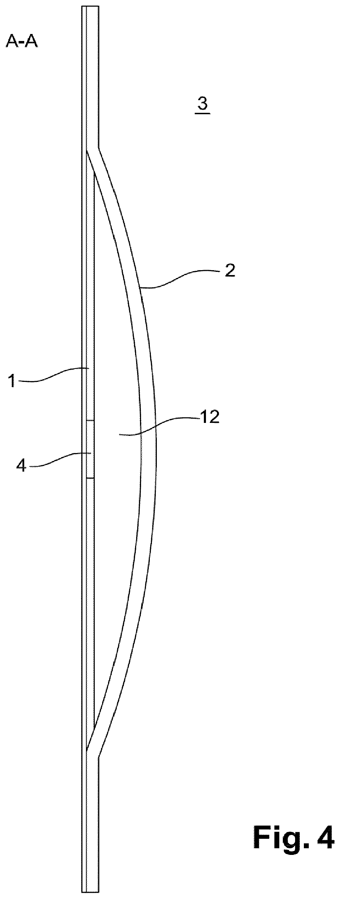

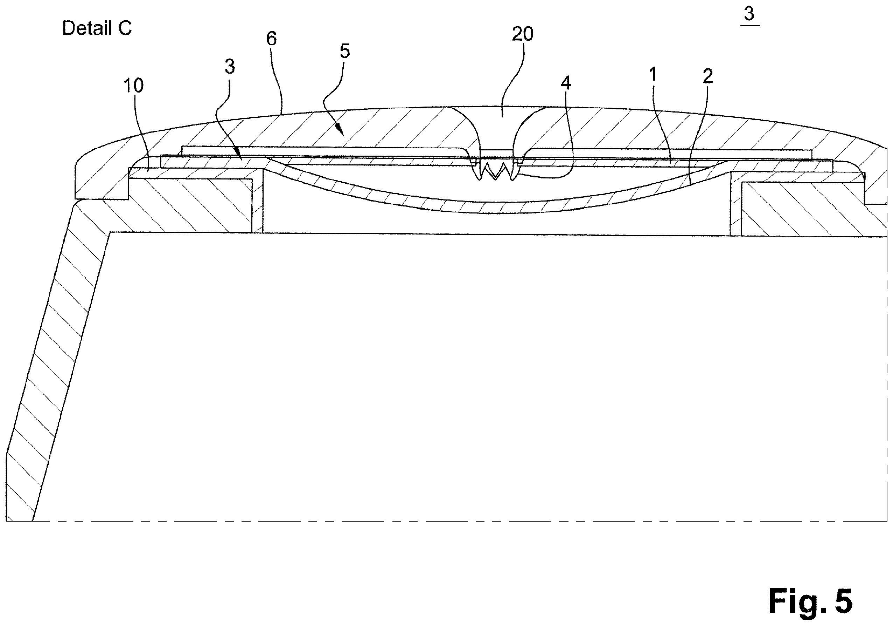

The structure of the reservoir 3 will now be described in greater detail with reference to Figures 1, 4 and 5.

The reservoir 3 comprises a first, electrically conductive zone 1 connected to the power supply system, and a second, elastically deformable zone 2. The second zone 2 initially bulges and flattens while the reservoir 3 is emptied in order to release the composition towards the application surface 5. The first zone 1 and the second zone 2 may be adhesively bonded or welded together. Together, they define a variable-volume space 12 for housing the composition. The space 12 may be in the form of a portion of sphere, as shown.

The second zone 2 may be in contact with the electrode 10, as shown, or not. If the second zone 2 is made of deformable and conductive material, it is necessary for it to be in contact with the electrode 10.

In order to start the treatment, the user turns on the device 1 by means of a push button 25. An electric current then starts to flow between the electrode 10 and the counter electrode 15 when the appliance is applied to the face for example, at the same time as the composition is dispensed.

In a variant embodiment, the intensity of the current may be adjusted by the user by virtue of an adjustment member 26 for adjusting the current density between the electrodes.

The treatment stops when the single-dose reservoir 3 is empty. The used-up reservoir 3 is removed in order to be replaced with a new reservoir in preparation for a new treatment.

The invention is not limited to the examples illustrated. In particular, the features of the various exemplary embodiments which have just been described can be combined with one another.

## Claims

1. Device (1) for cosmetically treating human keratin materials with the aid of an electric current, comprising at least:
- a power supply system,
- an electrode (10) and a counter electrode (15),
- a removable reservoir (3) containing a cosmetic composition, the reservoir (3) being provided with at least one outlet orifice (4) for the composition,
- an application end piece (6) that is fed with composition from the reservoir (3) and defines an application surface (5) intended to come into contact with the keratin materials,
**characterized in that** the reservoir (3) is at least partially defined by a first, electrically conductive zone (1) that is connected to the power supply system, and by a second, elastically deformable zone (2), the second, elastically deformable zone (2) exerting a pressure on the composition, this pressure being able to bring about a decrease in volume of the reservoir (3) and a flow of the composition through the outlet orifice (4) in the opened-up position of the outlet orifice (4), and the composition being kept inside the reservoir (3) in the closed-off position of the outlet orifice (4).

2. Device according to Claim 1, **characterized in that** the reservoir is defined by a maximum volume of between 50 microlitres, µl, and 10 ml.

3. Device according to either one of the preceding claims, **characterized in that** the cosmetic composition has a viscosity, measured at 25°C and at an atmospheric pressure of 1.013 10⁵ Pa, of less than 0.5 Pa.s.

4. Device according to any one of the preceding claims, **characterized in that** the application end piece (6) for the composition is conductive or non-conductive.

5. Device according to any one of the preceding claims, **characterized in that** it comprises a system for controlling the flow rate of the composition emerging from the reservoir (3) to between 1 µl/cm² and 50 µl/cm².

6. Device according to any one of the preceding claims, **characterized in that** each outlet orifice (4) defines an outlet cross-sectional area for the composition of less than 10 mm², preferably between 0.03 mm² and 10 mm².

7. Device according to any one of the preceding claims, **characterized in that** the first, electrically conductive zone (1) forms an electrode.

8. Device according to any one of the preceding claims, **characterized in that** the first, electrically conductive zone (1) is remote from the application surface (5).

9. Device according to any one of the preceding claims, **characterized in that** the first, electrically conductive zone (1) comprises a conductive coating or a conductive material.

10. Device according to any one of the preceding claims, **characterized in that** the second, elastically deformable zone (2) comprises an elastomer.

11. Device according to any one of the preceding claims, **characterized in that** the second, elastically deformable zone (2) comprises a material that has a level of elongation of at least 2%.

12. Device according to any one of the preceding claims, **characterized in that**, in the closed-off position of the outlet orifice (4), the reservoir (3) comprises a frangible zone that is able to tear under the effect of an action by the user.

13. Device according to any one of the preceding claims, **characterized in that** the reservoir (3) comprises an external layer of conductive hydrogel that is able to stabilize the pH.

14. Device according to any one of the preceding claims, **characterized in that** it comprises a source of heat, light, for setting the application end piece (6) in motion, notably a sonic, ultrasonic, radiofrequency or electroporation vibration.

15. Device according to any one of the preceding claims, **characterized in that** the power supply system delivers an alternating current with frequencies of, preferably, between 1 Hz and 5 MHz.

## Patentansprüche

1. Vorrichtung (1) zur kosmetischen Behandlung von menschlichen Keratinmaterialien mithilfe von elektrischem Strom, umfassend mindestens:
- ein Stromversorgungssystem,
- eine Elektrode (10) und eine Gegenelektrode (15),
- einen entfernbaren Behälter (3), der eine Kosmetikzusammensetzung enthält, wobei der Behälter (3) mit mindestens einer Auslassöffnung (4) für die Zusammensetzung ausgestattet ist,
- ein Applikationsendstück (6), das mit Zusammensetzung aus dem Behälter (3) gespeist wird und eine Applikationsoberfläche (5) definiert, die in Kontakt mit den Keratinmaterialien kommen soll,
**dadurch gekennzeichnet, dass** der Behälter (3) mindestens teilweise durch eine erste, elektrisch leitfähige Zone (1), die mit dem Stromversorgungssystem verbunden ist, und eine zweite, elastisch verformbare Zone (2) definiert ist, wobei die zweite, elastisch verformbare Zone (2) einen Druck auf die Zusammensetzung ausübt, wobei dieser Druck in der Lage ist, eine Abnahme des Volumens des Behälters (3) und einen Durchfluss der Zusammensetzung durch die Auslassöffnung (4) in der geöffneten Position der Auslassöffnung (4) zu bewirken, und wobei die Zusammensetzung in der verschlossenen Position der Auslassöffnung (4) im Inneren des Behälters (3) gehalten wird.

2. Vorrichtung nach Anspruch 1, **dadurch gekennzeichnet, dass** der Behälter durch ein Maximalvolumen zwischen 50 Mikrolitern (µl) und 10 ml definiert ist.

3. Vorrichtung nach einem der vorhergehenden Ansprüche, **dadurch gekennzeichnet, dass** die Kosmetikzusammensetzung eine bei 25 °C und einem atmosphärischen Druck von 1,013·10⁵ Pa gemessene Viskosität von weniger als 0,5 Pa·s aufweist.

4. Vorrichtung nach einem der vorhergehenden Ansprüche, **dadurch gekennzeichnet, dass** das Applikationsendstück (6) für die Zusammensetzung leitfähig oder nicht-leitfähig ist.

5. Vorrichtung nach einem der vorhergehenden Ansprüche, **dadurch gekennzeichnet, dass** sie ein System zur Steuerung der Durchflussrate der Zusammensetzung, die aus dem Behälter (3) austritt, auf zwischen 1 µl/cm² und 50 µl/cm² umfasst.

6. Vorrichtung nach einem der vorhergehenden Ansprüche, **dadurch gekennzeichnet, dass** jede Auslassöffnung (4) eine Auslassquerschnittsfläche für die Zusammensetzung von weniger als 10 mm² definiert, vorzugsweise zwischen 0,03 mm² und 10 mm².

7. Vorrichtung nach einem der vorhergehenden Ansprüche, **dadurch gekennzeichnet, dass** die erste, elektrisch leitfähige Zone (1) eine Elektrode bildet.

8. Vorrichtung nach einem der vorhergehenden Ansprüche, **dadurch gekennzeichnet, dass** die erste, elektrisch leitfähige Zone (1) sich entfernt von der Applikationsoberfläche (5) befindet.

9. Vorrichtung nach einem der vorhergehenden Ansprüche, **dadurch gekennzeichnet, dass** die erste, elektrisch leitfähige Zone (1) eine leitfähige Beschichtung oder ein leitfähiges Material umfasst.

10. Vorrichtung nach einem der vorhergehenden Ansprüche, **dadurch gekennzeichnet, dass** die zweite, elastisch verformbare Zone (2) ein Elastomer umfasst.

11. Vorrichtung nach einem der vorhergehenden Ansprüche, **dadurch gekennzeichnet, dass** die zweite, elastisch verformbare Zone (2) ein Material umfasst, das einen Dehnungsgrad von mindestens 2 % hat.

12. Vorrichtung nach einem der vorhergehenden Ansprüche, **dadurch gekennzeichnet, dass** der Behälter (3) in der verschlossenen Position der Auslassöffnung (4) eine zerbrechliche Zone umfasst, die unter Einwirkung einer Aktion seitens des Benutzers reißen kann.

13. Vorrichtung nach einem der vorhergehenden Ansprüche, **dadurch gekennzeichnet, dass** der Behälter (3) eine externe Schicht aus leitfähigem Hydrogel umfasst, das in der Lage ist, den pH-Wert zu stabilisieren.

14. Vorrichtung nach einem der vorhergehenden Ansprüche, **dadurch gekennzeichnet, dass** sie eine Quelle für Wärme, Licht umfasst, um das Applikationsendstück (6) in Bewegung zu setzen, insbesondere eine Schall-, Ultraschall-, Hochfrequenz- oder Elektroporationsvibration.

15. Vorrichtung nach einem der vorhergehenden Ansprüche, **dadurch gekennzeichnet, dass** das Stromversorgungssystem einen Wechselstrom mit Frequenzen von vorzugsweise zwischen 1 Hz und 5 MHz liefert.

## Revendications

1. Dispositif (1) destiné à traiter cosmétiquement des matières kératiniques humaines à l'aide d'un courant électrique, comprenant au moins :
- un système d'alimentation électrique,
- une électrode (10) et une contre-électrode (15),
- un réservoir amovible (3) contenant une composition cosmétique, le réservoir (3) étant pourvu d'au moins un orifice de sortie (4) pour la composition,
- un embout d'application (6) qui est alimenté avec une composition depuis le réservoir (3) et définit une surface d'application (5) destinée à venir en contact avec les matières kératiniques,
**caractérisé en ce que** le réservoir (3) est au moins partiellement défini par une première zone, électriquement conductrice (1) qui est reliée au système d'alimentation électrique, et par une deuxième zone, élastiquement déformable (2), la deuxième zone, élastiquement déformable (2) exerçant une pression sur la composition, cette pression pouvant provoquer une diminution de volume du réservoir (3) et un écoulement de la composition à travers l'orifice de sortie (4) dans la position ouverte de l'orifice de sortie (4), et la composition étant maintenue à l'intérieur du réservoir (3) dans la position fermée de l'orifice de sortie (4).

2. Dispositif selon la revendication 1, **caractérisé en ce que** le réservoir est défini par un volume maximal compris entre 50 microlitres, µl, et 10 ml.

3. Dispositif selon l'une ou l'autre des revendications précédentes, **caractérisé en ce que** la composition cosmétique a une viscosité, mesurée à 25 °C et à une pression atmosphérique de 1,013.10⁵ Pa, de moins de 0,5 Pa.s.

4. Dispositif selon l'une quelconque des revendications précédentes, **caractérisé en ce que** l'embout d'application (6) pour la composition est conducteur ou non conducteur.

5. Dispositif selon l'une quelconque des revendications précédentes, **caractérisé en ce qu'**il comprend un système destiné à contrôler le débit de la composition sortant du réservoir (3) entre 1 µl/cm² et 50 µl/cm².

6. Dispositif selon l'une quelconque des revendications précédentes, **caractérisé en ce que** chaque orifice de sortie (4) définit une aire de section droite de sortie pour la composition de moins de 10 mm², de préférence entre 0,03 mm² et 10 mm².

7. Dispositif selon l'une quelconque des revendications précédentes, **caractérisé en ce que** la première zone, électriquement conductrice (1) forme une électrode.

8. Dispositif selon l'une quelconque des revendications précédentes, **caractérisé en ce que** la première zone, électriquement conductrice (1) est distante de la surface d'application (5).

9. Dispositif selon l'une quelconque des revendications précédentes, **caractérisé en ce que** la première zone, électriquement conductrice (1) comprend un revêtement conducteur ou un matériau conducteur.

10. Dispositif selon l'une quelconque des revendications précédentes, **caractérisé en ce que** la deuxième zone, élastiquement déformable (2) comprend un élastomère.

11. Dispositif selon l'une quelconque des revendications précédentes, **caractérisé en ce que** la deuxième zone, élastiquement déformable (2) comprend un matériau qui a un niveau d'allongement d'au moins 2 %.

12. Dispositif selon l'une quelconque des revendications précédentes, **caractérisé en ce que**, dans la position fermée de l'orifice de sortie (4), le réservoir (3) comprend une zone de rupture qui peut se déchirer sous l'effet d'une action de l'utilisateur.

13. Dispositif selon l'une quelconque des revendications précédentes, **caractérisé en ce que** le réservoir (3) comprend une couche externe d'hydrogel conducteur qui peut stabiliser le pH.

14. Dispositif selon l'une quelconque des revendications précédentes, **caractérisé en ce qu'**il comprend une source de chaleur, de lumière, pour mettre l'embout d'application (6) en mouvement, notamment une vibration sonore, ultrasonore, radiofréquence ou d'électroporation.

15. Dispositif selon l'une quelconque des revendications précédentes, **caractérisé en ce que** le système d'alimentation électrique délivre un courant alternatif avec des fréquences de préférence comprises entre 1 Hz et 5 MHz.
